# EUROPÄISCHE PATENTSCHRIFT

(11) **EP 4 265 188 B1**
(45) Veröffentlichungstag und Bekanntmachung des Hinweises auf die Patenterteilung: **12.02.2025**
(21) Anmeldenummer: 22197506.3
(22) Anmeldetag: 23.09.2022
(51) Int. Cl.: A61B 6/03

(54) **DREHGESTELL FÜR DIE GANTRY EINES COMPUTERTOMOGRAFEN, GANTRY UND COMPUTERTOMOGRAF**
ROTATING STRUCTURE FOR THE GANTRY OF A COMPUTER TOMOGRAPH, GANTRY AND COMPUTER TOMOGRAPH
BOGIE POUR PORTIQUE DE TOMOGRAPHIE ASSISTÉE PAR ORDINATEUR, PORTIQUE ET APPAREIL DE TOMOGRAPHIE ASSISTÉE PAR ORDINATEUR

(43) Veröffentlichungstag der Anmeldung: 25.10.2023
(73) Patentinhaber: Siemens Healthineers AG, 91301 Forchheim (DE)
(72) Erfinder: Müller, Hans-Jürgen, 91362 Pretzfeld (DE)
(74) Vertreter: Siemens Healthineers Patent Attorneys

(56) Entgegenhaltungen:
- DE-A1- 102016 208 328
- DE-U1- 202021 106 779

## Beschreibung

Die Erfindung betrifft ein Drehgestell für die Gantry eines Computertomografen, eine Gantry für einen Computertomografen und einen Computertomografen.

DE 10 2016 208328 A1 offerbart eine bionisch inspirierte Gehäusestruktur für Gantry, hergestellt im 3D-Druckverfahren. DE 20 2021 106779 U1 offerbart eine 3D-gedruckte CT-Gantry die Aluminiumteile in der Gehäusestruktur enthält. Bei der Computertomografie (CT) werden Schnittbilder des Patienten aus Röntgenprojektionen aus einer Vielzahl von Projektionsrichtungen rekonstruiert. Zur Aufnahme der Röntgenprojektionen aus verschiedenen Projektionsrichtungen rotieren eine Röntgenquelle und ein ihr gegenüber liegender Röntgendetektor um einen Aufnahmebereich. Beim Spiral-CT wird der zu untersuchende Patient zeitgleich auf einer Liege durch den Tunnel einer Gantry geführt. Alle rotierenden Komponenten, insbesondere die Röntgenquelle und der Röntgendetektor, werden beim Aufbau eines Computertomografen an einem Drehgestell befestigt, welches drehbar in der Gantry des Computertomografen gelagert ist. Ein solches Drehgestell wird auch als Drehrahmen, Drum oder drehbarer Teil der Gantry bezeichnet. Die Gantry weist ferner typischerweise einen festen Tragrahmen auf, an dem Verkleidungselemente befestigt sind. In diesem Tragrahmen ist das Drehgestell drehbar gelagert, beispielsweise mittels eines Wälzlagers oder Magnetlagers.

Um die Strahlungsbelastung für den Patienten so weit wie möglich zu reduzieren, und zur Vermeidung von Bewegungsartefakten, ist man bestrebt, eine möglichst hohe Rotationsgeschwindigkeit von Röntgenquelle und Röntgendetektor zu ermöglichen, beispielsweise bis zu 240 U/min. Deshalb muss das Drehgestell höchsten Anforderungen in Sachen Steifigkeit genügen, damit sich das Drehgestell auch bei hohen Rotationsgeschwindigkeiten nicht verformt. Leichte Verformungen von nur wenigen Zehntel Millimetern können bereits schwere Bildartefakte zur Folge haben, wenn die relative Position von der Röntgenquelle zum Röntgendetektor nicht exakt konstant bleibt. Im Stand der Technik ist das Drehgestell daher als ein Aluminium- oder Stahlgussteil mit einer relativ hohen Wandstärke von ca. 20-25 mm ausgebildet. Ein solches Gussteil liefert die erforderliche Steifigkeit und ein hohes Widerstandsmoment gegen Belastung. Darüber hinaus weist das Drehgestell im Stand der Technik typischerweise eine Topf- oder Trommelform auf, sodass die rotierenden Komponenten auch bei hohen Rotationsgeschwindigkeiten stabil gehalten werden. Ein derartiges Drehgestell es beispielsweise in der DE 10 2013 227 060 A1 offenbart.

Bei jeder Untersuchung wird das Drehgestell mitsamt der Röntgenquelle und weiteren rotierenden Komponenten möglichst schnell auf die für die Untersuchung benötigte Rotationszeit beschleunigt, was entsprechend Energie auf Seiten des Antriebsmotors des Drehgestells benötigt.

Der Erfindung liegt die Aufgabe zugrunde, ein Drehgestell mit einer Alternative zu bekannten Befestigungsmöglichkeiten für die rotierenden Komponenten des Computertomografen bereitzustellen.

Diese Aufgaben werden gelöst durch ein Drehgestell nach Anspruch 1, durch eine Gantry nach Anspruch 12, sowie durch einen Computertomographen nach Anspruch 13.

Das erfindungsgemäße Drehgestell weist Aufnahmebereiche auf, an denen rotierende Komponenten des Computertomografen befestigbar sind und ist dadurch gekennzeichnet, dass es zumindest teilweise durch additive Fertigung hergestellt ist. Unter additiver Fertigung, auch 3-D Druck genannt, werden insbesondere Herstellungsverfahren verstanden, bei denen Schicht für Schicht Material aufgetragen wird, um dreidimensionale Werkstücke herzustellen. Dies geschieht typischerweise computergesteuert aus flüssigen oder pulverförmigen Materialien gemäß einer CAD Planung, sodass beliebige dreidimensionale (3-D) Formen realisierbar sind.

Durch die zumindest teilweise Herstellung mittels additiver Fertigung ist es möglich, die Struktur des Drehgestells in Richtung Leichtbau bei gleichzeitig maximaler Formsteifigkeit zu optimieren. Es kann also zum Beispiel ein 3-D gedrucktes erfindungsgemäßes Drehgestell mit einem strukturoptimierten Design bereitgestellt werden. Durch die große Gestaltungsfreiheit in der Formgebung des Drehgestells ist es möglich, ein geringes Gewicht, insbesondere durch geringe Wandstärken und/oder die Verwendung von Material mit geringem spezifischen Gewicht, zu realisieren, und trotzdem noch genügend Steifheit bzw. Formsteifigkeit für die geforderten hohen Drehzahlen, oder sogar für noch höhere Drehzahlen als zurzeit üblich, bereitzustellen. Insbesondere kann das erfindungsgemäße Drehgestell für Rotationsgeschwindigkeiten von über 260 U/min, vorzugsweise etwa 280-320 U/min geeignet sein.

Die Gewichtsreduktion des Drehgestells hat ferner den Vorteil, dass auf das Rotationslager der Gantry eine geringere statische Last wirkt. Ferner reduziert sich das Gesamtgewicht der Gantry und somit auch die Transportkosten und die Bodenbelastung beim Kunden. Vor allem wird weniger Rotations- und Beschleunigungsenergie benötigt, da das Gewicht der rotierenden Einheit (Drehgestell und rotierende Komponenten) verringert wird. Dadurch ist ein kleinerer Rotationsmotor möglich und/oder eine schnellere Beschleunigung auf die Nenndrehzahl, was sich wiederum in einer kürzeren Wartezeit für Patienten und einen höheren Patientendurchsatz niederschlägt.

Bei der Herstellung des Drehgestells durch additive Fertigung kann die Topologie bzw. Struktur des Drehgestells durch einen Algorithmus optimiert werden. Dadurch können auch unkonventionelle, beispielsweise nicht rotationssymmetrische Topologie berechnet werden, die bei einer vorgegebenen hohen Drehzahl zu erwartenden Belastungen standhält. Bei der Berechnung können auch Sicherungselemente vorgegeben werden, beispielsweise damit im Fall eines lokalen Risses des Drehgestells in kein vollständiges Versagen auftritt.

Unter "rotierende Komponenten" des Computertomografen werden alle diejenigen Komponenten verstanden, die bei der Akquisition der CT-Daten um den Patienten rotieren, insbesondere die Röntgenquelle und der Röntgendetektor. Typischerweise sind die rotierenden Komponenten im aufgestellten CT-Gerät am Drehgestell befestigt. Weitere rotierende Komponenten können zum Beispiel vor der Röntgenröhre angebrachte Filter und Blenden, eine Strahlerkühlung, ein Spannungsgenerator und weitere zum Betrieb von Röntgenröhre und Röntgendetektor notwendige elektronische Geräte sein, soweit diese nicht stationär außerhalb des Drehgestells angeordnet werden können, sondern direkt mit Röntgenquelle und Röntgendetektor verbunden sein müssen, damit diese betrieben werden können. Unter einem Aufnahmebereich für eine rotierende Komponente wird der Bereich verstanden, in dem diese Komponente im zusammengebauten CT-Gerät angeordnet und am Drehgestell befestigt sein soll.

Das Drehgestell kann zumindest teilweise mittels einem der folgenden additiven Fertigungsverfahren hergestellt sein: Fused Deposition Modeling (FDM), Selektives Laserschmelzen (SLM), Selektives Lasersintern (SLS), Elektronenstrahlschmelzen (EBM), Directed Energy Deposition (DED).

Ein bevorzugtes Verfahren ist das DED-Verfahren, da es maximale Gestaltungsfreiheit liefert und gleichzeitig für viele Materialien, darunter auch Metalle, geeignet ist. Zudem eignet es sich auch für Hybridanwendungen, also zum Beispiel zum Ansetzen von Elementen an bereits vorhandene Werkstücke. Beim DED kann ein Hochleistungslaser verwendet werden, um Metallpulver zu schmelzen, das dem Fokus des Laserstrahls zugeführt wird. Der Laserstrahl verläuft in der Regel durch die Mitte eines Beschichtungskopfes und wird durch eine oder mehrere Linsen auf einen kleinen Punkt fokussiert. Das Metallpulver wird über den Umfang des Beschichtungskopfes zugeführt und verteilt oder kann durch Düsen, die um den Beschichtungskopf herum angeordnet sind, zugeführt werden. Der Aufbau erfolgt auf einem in einer Ebene beweglichen Tisch, der auf einer Spur bewegt wird, die anhand eines digitalen Modells erstellt wurde, um ein Objekt Schicht für Schicht herzustellen. Der Beschichtungskopf wird vertikal nach oben bewegt, wenn jede Schicht fertiggestellt ist. Einige Systeme verwenden sogar 5-Achsen oder 6-Achsen-Systeme (d. h. Gelenkarme), die in der Lage sind, Material auf das Werkstück mit wenigen bis keinen räumlichen Zugangsbeschränkungen aufzubringen. Das DED-Verfahren eignet sich für eine breite Palette von Werkstoffen wie Titan, Edelstahl, Aluminium, Wolfram und andere Spezialwerkstoffe sowie für Verbundwerkstoffe. Mit dem Verfahren können nicht nur neue Werkstücke vollständig hergestellt werden, sondern es kann auch Material zu bestehenden Teilen hinzugefügt werden, zum Beispiel für hybride Fertigungsanwendungen.

Auch das selektive Lasersintern (SLS) ist sehr geeignet. Dabei wird ein Laser als Wärmequelle verwendet wird, um pulverförmiges Material (z.B. Nylon oder Polyamid) zu sintern, wobei der Laser automatisch auf Punkte im Raum gerichtet wird, die durch ein 3-D-Modell definiert sind, wobei der Laser das Material zu einer festen Struktur verbindet. Es gibt hierfür schnelle 3-D Druck Verfahren, bei welchen das Werkstück jedoch nach dem Drucken noch gesintert wird, um eine ausreichend feste und steife Verbindung zu erhalten.

Möglich sind ferner additive Fertigungsmethoden für Kunststoffe, bei denen das Drehgestell zumindest zum Teil aus faserverstärktem Kunststoff aufgebaut wird.

Das Drehgestell kann zumindest teilweise aus einem der Werkstoffe ausgewählt aus Stahl, einer Stahllegierung, Aluminium, einer Aluminiumlegierung, faserverstärktem Kunststoff, einer Titanlegierung, einer Chrom-Kobaltlegierung oder einer Magnesiumlegierung gefertigt sein. Vorzugsweise ist es zumindest teilweise aus einem Werkstoff gefertigt, der sich für additive Fertigung eignet und/oder relativ leicht ist, wie zum Beispiel Aluminium und Aluminiumlegierungen und/oder faserverstärkter Kunststoff (FVK), insbesondere glasfaserverstärkter Kunststoff (GFK) oder kohlefaserverstärkter Kunststoff (CFK). GFK oder CFK kann mittels additiver Fertigung so verarbeitet werden, dass die Fasern in der Kraftrichtung verlaufen, so dass diese besonders für die Herstellung von Verstärkungselementen, Verstrebungen oder Fachwerkelementen in Leichtbauweise geeignet sind.

Gemäß einer Ausführungsform weist das Drehgestell mittels additiver Fertigung hergestellte Verstrebungen auf. Eine Verstrebung ist z. B. eine Strebe, Balken oder Fachwerkelement, welches zwei Punkte des Drehgestells kraftschlüssig miteinander verbindet. Insbesondere kann eine Verstrebung Kräfte entlang ihrer Längsrichtung aufnehmen. Zwischen der Verstrebung und anderen Bereichen des Drehgestells können eine oder mehrere Aussparungen liegen. Durch Verstrebungen kann eine besonders hohe Formsteifigkeit bei möglichst geringem Gewicht erzielt werden, sie sind aber durch die herkömmlichen Herstellungsverfahren des Drehgestells, wie Aluminium-Sandguss, nicht leicht herzustellen. Dies gilt vor allem, wenn die Verstrebungen in mehreren Ebenen vorgesehen sein sollen. Mittels additiver Fertigung hingegen können Verstrebungen in beliebigen Formen und Richtungen gefertigt werden.

Gemäß einer bevorzugten Ausführungsform weist das Drehgestell zumindest eine mittels additiver Fertigung hergestellte Verstrebung auf, die zwei Punkte auf einem um die Achse angeordneten ringförmigen Element, insbesondere am Außenumfang des Drehgestells, miteinander verbindet, insbesondere nach Art einer Sehne, wobei die Verstrebung nicht gerade sein muss. Die Verstrebung kann z. B. nach außen, von der Achse aus gesehen, gewölbt sein. Durch eine derartige Verstrebung kann auch bei hohen Drehzahlen eine große Formsteifigkeit des Drehgestells vor allem an dessen Außenumfang erreicht werden. Vorteilhaft sind zwei bis drei solcher Verstrebungen vorgesehen, die z. B. jeweils Punkte in einem Winkelabstand von 90 bis 170 Grad miteinander verbinden.

Das Drehgestell ist typischerweise um eine Achse drehbar, welche im aufgestellten CT-Gerät meist horizontal durch den Mittelpunkt der Gantry verläuft. Die rotierenden Komponenten des CT-Geräts können ungefähr ringförmig um diese Achse angeordnet sein. Zu diesem Zweck weisen herkömmliche, aus Aluminium-Sandguss hergestellte Drehgestelle oft eine Grundplatte auf, welche die Grundform einer runden Lochscheibe hat und am Außenumfang in eine etwa zylinderförmige Trommel übergeht, welche die rotierenden Komponenten in Umfangsrichtung umschließt. Ein Gussteil kann jedoch, ausgehend von der Grundplatte, keine Hinterschneidungen aufweisen, da sonst mit einer verlorenen Form gearbeitet werden müsste. Die Trommel kann sich also nicht wieder nach innen wölben, um z. B. einen der rotierenden Komponenten von zwei Seiten zu halten.

Das erfindungsgemäße Drehgestell weist zumindest eine mittels additiver Fertigung ausgebildete Hinterschneidung auf. Dies kann bedeuten, dass sich Abschnitte des Drehgestells in Axialrichtung verdicken und/oder wölben, jedenfalls nicht innerhalb ihres Fußabdrucks in Axialrichtung bleiben. Eine Hinterschneidung kann auch ein Abschnitt des Drehgestells sein, welches einen Aufnahmebereich für eine rotierende Komponente des Computertomographen zumindest teilweise umschließt, so dass dieser insbesondere von zwei gegenüberliegenden Seiten umschlossen wird.

Die mittels additiver Fertigung ausgebildete Hinterschneidung trägt dazu bei, dass ein solcher Aufnahmebereich von zwei Seiten von Abschnitten des Drehgestells zumindest teilweise umgriffen wird, was eine besonders stabile Befestigung der rotierenden Komponente ermöglicht. Insbesondere kann vorgesehen sein, dass der Aufnahmebereich in Axialrichtung und/oder in Umfangsrichtung und/oder in Radialrichtung von den zwei Seiten von den Abschnitten des Drehgestells umgeben sein kann, besonders bevorzugt in Axialrichtung und/oder in Radialrichtung.

Das Drehgestell kann die Form eines Hohlkörpers aufweisen, bei welchem insbesondere Aufnahmebereiche von Wandungen und/oder Verstrebungen des Drehgestells umgeben sein können.

Insbesondere kann das Drehgestell ungefähr die Form eines nach innen offenen Torus ausweisen, wobei die Außenfläche des Torus vorzugsweise mit Aussparungen versehen und/oder zumindest teilweise von Verstrebungen oder Fachwerk gebildet ist. Von innen, also von der Achse aus, können die rotierenden Komponenten in das Drehgestell eigesetzt werden. Der Torus muss nicht von einem Kreis aufgespannt werden, sondern kann im Querschnitt auch eine annähernd ovale, quadratische, parallelogrammförmige oder rechteckige Form, ggf. mit abgerundetem Ecken, aufweisen. Ein solcher im Wesentlichen von Verstrebungen oder Fachwerk gebildeter Körper wird auch als "Vogelnest" bezeichnet.

Das Drehgestell kann auch die Form eines hohlen Ringes oder Reifens aufweisen, wobei auch diese Form vorzugsweise mit Aussparungen versehene Wandungen und/oder Verstrebungen oder Fachwerk aufweist.

Gemäß einer Ausführungsform ist das Drehgestell in Leichtbauweise ausgeführt, insbesondere mit einer mittleren Wandstärke von bis zu 30mm, vorzugsweise 5mm bis 20mm, stärker bevorzugt 10-18mm. Durch die additive Fertigung ist es möglich, die Struktur des Drehgestells derart zu optimieren, dass seinen Wandungen und Verstrebungen mit einer sehr geringen Wandstärke ausgeführt sein können, ohne Steifigkeit einzubüßen. Dazu können zum einen die Verstrebungen, zum andere die optimierte Grundform, wie z. B. die Reifen- bzw. Vogelnestform beitragen.

Gemäß einer Ausführungsform wiegt das Drehgestell 20%-60%, vorzugsweise 30%-50%, weniger als ein entsprechendes Drehgestell aus Aluminiumguss gemäß Stand der Technik.

Gemäß einer Ausführungsform wiegt das Drehgestell weniger als 200kg, vorzugsweise weniger als 160 kg, stärker bevorzugt 100-140kg. Diese Angaben beziehen sich auf ein Drehgestell mit einem Innendurchmesser von 60-100cm, vorzugsweise 70-90 cm, insbesondere 80mm, und einem Außendurchmesser von 1500mm bis 18000mm, insbesondere 1600mm bis 1700mm, z.B. 1650mm, und ohne die rotierenden Komponenten. Ein konventionell mit Aluminium-Sandguss hergestelltes Drehgestell dieser Größe hat dagegen eine Masse von ca. 240kg. Bei kleineren Drehgestellen z. B. für Kopf-Computertomografen besteht ein prozentual ähnlich großes Gewichtseinsparpotential. Die durch die additive Fertigung und die optimierte Struktur erreichbare Gewichtseinsparung ist also erheblich.

Gemäß einer Ausführungsform weist das Drehgestell einen nicht durch additive Fertigung hergestellten Rahmen und weitere, durch additive Fertigung hergestellte Elemente auf. Dadurch können die Vorteile von konventionellen und additiven Herstellungsverfahren vorteilhaft miteinander kombiniert werden. Beispielsweise ist es möglich, einen Rahmen kostengünstig z. B. aus einem gewalzten Metallelement, insbesondere einer gewalzten flachen Platte aus Aluminiumlegierung oder Stahl, herzustellen. Der Rahmen kann z. B. die Grundform einer runden Lochscheibe aufweisen, die aus einem gewalzten Metallprodukt ausgestanzt ist. Alternativ kann der Rahmen auch ein Gussteil aus Metall oder Kunststoff sein, oder aus einem stranggegossenen Metallelement gefertigt. Schließlich kann der Rahmen auch aus einem zylinderförmigen rundgewalzten oder stranggegossenen Metallteil gefertigt sein. Der Rahmen kann zusätzlich mechanisch bearbeitet sein, z.B. durch Drehen oder Fräsen, beispielsweise um Aussparungen zu realisieren.

Gemäß einer Ausführungsform der Erfindung sind an einem solchen "Grundrahmen" weitere durch additive Fertigung hergestellte Elemente befestigt, die insbesondere zur Stabilisierung und Formsteifigkeit des Drehgestells beitragen. Dies kann dadurch geschehen, dass die weiteren Elemente getrennt durch additive Fertigung hergestellt werden und dann mit dem Rahmen verbunden, z. B. durch Schweißen, Kleben, durch Formschluss, durch Verbindungselemente wie Schrauben oder Nieten, oder einer Kombination dieser Verbindungen. Das hat den Vorteil, dass man die weiteren Elemente besonders kostengünstig herstellen kann und dass man in ihrer Formgebung besonders viele Freiheitsgerade hat. Die verbundenen Elemente sind insbesondere Verstrebungen. Es ist auch möglich, die weiteren Elemente direkt mit additiver Fertigung an den Rahmen anzusetzen. Das hat den Vorteil, dass man sich einen weiteren Verbindungsschritt spart. In diesem Fall kann der Rahmen in das Werkzeug für die additive Fertigung eingespannt werden und die weiteren Elemente direkt auf den Grundrahmen z. B**.** durch DED aufgebaut werden.

Gemäß einer alternativen Ausführungsform ist das Drehgestell vollständig durch additive Fertigung hergestellt. Dies erlaubt eine sehr weitgehende Optimierung der Konstruktion. Das Drehgestell kann einstückig hergestellt sein, es kann aber auch aus mehreren miteinander verbundenen Elementen aufgebaut sein, wobei die einzelnen Elemente wiederum durch additive Fertigung hergestellt sind. Dies erlaubt die Kombination verschiedener Werkstoffe, insbesondere von Metall mit FVK, was eine besonders gewichtssparende Konstruktion erlaubt.

Gemäß einer Ausführungsform umfasst das Drehgestell mehrere miteinander verbundenen Elemente, von denen zumindest eines durch additive Fertigung hergestellt ist. Beispielsweise können ein oder mehrere durch additive Fertigung hergestellte Elemente wie Verstrebungen mit einem nicht durch additive Fertigung hergestelltem Rahmen verbunden sein, wie oben beschrieben. Vorteilhaft ist zumindest 20%, bevorzugt 35%, stärker bevorzugt 50% der Masse des Drehgestells durch additive Fertigung hergestellt.

Gemäß einer Ausführungsform weist das Drehgestell Fachwerkelemente auf, die zumindest einen Aufnahmebereich für eine rotierende Komponente des CT-Geräts auf zumindest einer Seite umschließen, vorzugsweise auf zwei oder drei Seiten. Das Fachwerk kann insbesondere ein Stabwerk sein, dessen Stäbe an ihren Enden an Knotenpunkten miteinander verbunden sind. Vorteilhaft werden die Stäbe überwiegend oder vollständig durch Kräfte entlang ihrer Längsrichtung beansprucht, insbesondere auf Zug. Dadurch kann bei geringem Materialeinsatz eine besonders hohe Formsteifigkeit des Drehgestells erreicht werden. Die Fachwerkelemente bzw. ein von diesen gebildetes Fachwerk kann eine Wandung des Drehgestells bilden, insbesondere eine Wandung an einer äußeren Umfangsfläche des Drehgestells, oder eine Wandung in Form einer flachen oder gewölbten Lochscheibe, welche etwa die Funktion der Grundplatte des herkömmlichen Drehgestells übernimmt.

Gemäß einer Ausführungsform weist das Drehgestell zumindest einen durchgehenden Ring auf, an dem bzw. an denen durch additive Fertigung hergestellte Elemente, insbesondere Verstrebungen, angesetzt sind. Der Ring kann ebenfalls durch additive Fertigung hergestellt sein, er kann auch herkömmlich zum Beispiel als Gussteil oder stranggepresstes und/oder gebogenes Teil gefertigt sein. Der Ring legt vorteilhaft den Innendurchmesser des Drehgestells fest. Seine Achse kann mit der Achse des Drehgestells übereinstimmen. An einem solchen inneren Ring kann sich im zusammengebauten CT-Gerät die Lagerung befinden, an der das Drehgestell drehbar gelagert ist. Von einem solchen inneren Ring können Verstrebungen insbesondere radial nach außen verlaufen, gegebenenfalls mit einer Wölbung hin zur Axialrichtung, um einen Aufnahmebereich für eine rotierende Komponente zu bilden.

Gemäß einer vorteilhaften Ausführungsform sind zumindest einige dieser Verstrebungen an ihrem anderen Ende wieder mit einem Ring verbunden, welcher insbesondere zu dem inneren Ring koaxial verläuft. Dieser Ring kann am äußeren Umfang des Drehgestells angeordnet sein, also in Radialrichtung außen. Dieser äußere Ring kann die rotierenden Komponenten außen umschließen, gegebenenfalls können rotierende Komponenten auch an dem äußeren Ring befestigt sein. Der äußere Ring kann in Axialrichtung gegenüber dem inneren Ring versetzt angeordnet sein. Gemäß einer Ausführungsform sind an dem äußeren Ring Verstrebungen angesetzt, die den äußeren Ring stabilisieren, insbesondere dessen Verformung bei hohen Drehzahlen verhindern. Dabei kann es sich um sehnenartige Verstrebungen handeln, die jeweils zwei in Umfangsrichtung um 90° bis 170° beabstandete Punkte auf dem äußeren Ring miteinander verbinden. Derartige sehnenartige Verstrebungen können wiederum mit weiteren Verstrebungen, welche zum äußeren Ring verlaufen, verbunden sein. Die sehnenartige Verstrebungen können einen Aufnahmebereich auf einer dem inneren Ring gegenüberliegenden Seite umschließen und somit ein sicheres Halten von rotierenden Komponenten ermöglichen.

Der innere und/oder äußere Ring des Drehgestells kann mittels additiver Fertigung hergestellt sein. Er kann aber auch durch ein konventionelles Verfahren, beispielsweise Biegen von Metallstangen oder Stanzen aus einem gewalzten Metall-Flachprodukt, hergestellt sein. Vorzugsweise sind die oben genannten Verstrebungen mittels additiver Fertigung hergestellt, insbesondere an den inneren und/oder äußeren Ring angesetzt.

Gemäß einer Ausführungsform weist das Drehgestell eine solche Steifigkeit auf, dass es bei einer Rotation im Betrieb des Computertomografen maximal bis zu 0,3 mm, vorzugsweise bis zu 0,2 mm, verformbar ist. Mit Rotation ist hier eine Rotationsgeschwindigkeit von mindestens 240 U/min, bevorzugt mindestens 300 U/min gemeint. Beiträge stellen gemäß Stand der Technik ist bei derartigen Drehzahlen eine erheblich höhere Verformung zu erwarten, insbesondere bis zu 0,6 mm oder sogar mehr.

Die Erfindung ist auch auf eine Gantry für einen Computertomografen mit einem Drehgestell wie hierin beschrieben gerichtet, an welchem rotierende Komponenten des Computertomografen, insbesondere eine Röntgenquelle und eine Röntgendetektoreinheit, befestigt sind. Die Gantry weist typischerweise übliche Maße auf, und kann neben dem Drehgestell und den darin eingesetzten rotierenden Komponenten auch ein Traggestell und eine Verkleidung aufweisen.

Gemäß einem weiteren Aspekt ist die Erfindung auch auf einen Computertomografen gerichtet, welcher ein erfindungsgemäßes Drehgestell und/oder eine erfindungsgemäße Gantry aufweist. Der Computertomograf kann ferner eine Gantry wie oben beschrieben aufweisen. Weitere Komponenten wie beispielsweise ein Rechner zur Rekonstruktion der akquirierten Daten und eine Bedienkonsole können ebenfalls Teil des Computertomografen sein. Alle mit Bezug auf das Drehgestell beschriebene Vorteile und Ausführungsbeispiele treffen auch auf die Gantry und den Computertomografen zu, und umgekehrt.

Die Erfindung wird nun anhand von Ausführungsbeispielen mit Bezug auf die beiliegenden Zeichnungen näher beschrieben. In den Zeichnungen zeigen:
- Fig. 1: eine schematische perspektivische Darstellung eines Computertomografen gemäß einem Ausführungsbeispiel der Erfindung;
- Fig. 2: eine schematische Draufsicht auf ein Drehgestell;
- Fig. 3: eine perspektivische Ansicht eines Drehgestell gemäß Stand der Technik;
- Fig. 4: eine perspektivische Ansicht eines Drehgestells gemäß einer Ausführungsform der Erfindung von einer ersten Seite;
- Fig. 5: eine perspektivische Ansicht eines Drehgestells gemäß Figur 4 von einer zweiten Seite;
- Fig. 6: eine schematische Schnittansicht einer weiteren Ausführungsform eines Drehgestells;
- Fig. 7: eine schematische Schnittansicht einer noch weiteren Ausführungsform eines Drehgestells.

Gleiche oder ähnliche Elemente sind in den Figuren mit den gleichen Bezugszeichen gekennzeichnet.

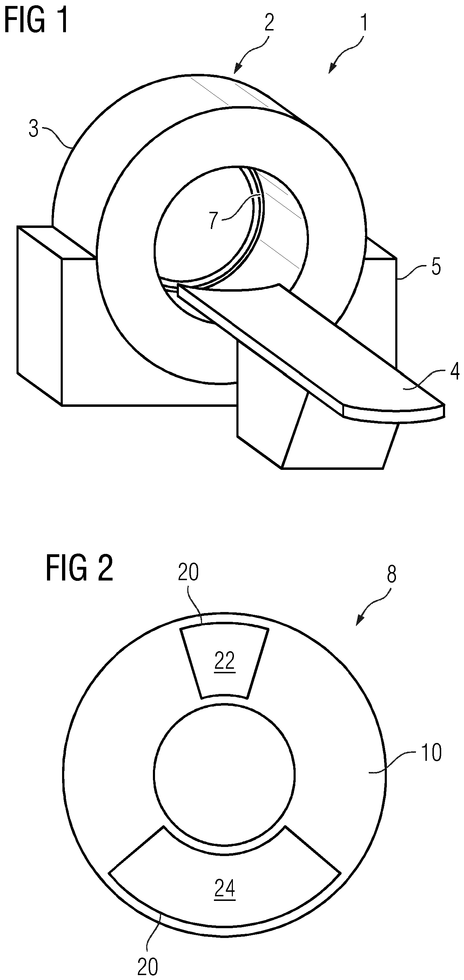

Figur 1 zeigt vereinfacht ein CT-Gerät 1 mit einer Gantry 2, in welcher eine erfindungsgemäßes Drehgestell 30 aufgenommen ist. Dieses ist auf einem Traggestell 5 gelagert. Die Gantry 2 ist mit einer Verkleidung 3 versehen. Sie weist eine ringförmige Öffnung auf, durch welche die Liege 4 vorgeschoben werden kann. Rings um die ringförmige Öffnung verläuft ein Ring 7, an welchem das Drehgestell gelagert ist, z. B. durch ein Wälzlager, dass es um einen auf der Liege 4 gelagerten Patienten rotieren kann. Der Antriebsmechanismus für die Rotation ist nicht dargestellt.

Figur 2 zeigt eine schematische Draufsicht auf ein Drehgestell 8 gemäß Stand der Technik. Das Drehgestell weist eine Grundplatte 10 auf, an welcher die rotierenden Komponenten 20 befestigt sind. Die rotierenden Komponenten des 20 Computertomografen sind insbesondere eine Röntgenröhre 22 und eine Röntgendetektionseinheit 24. Weitere Komponenten, die in Figur 2 nicht dargestellt sind, jedoch um den weiteren Umfang des Drehgestells 8 angeordnet sein können, sind beispielsweise Hochspannungsgeneratoren und Kühlsysteme.

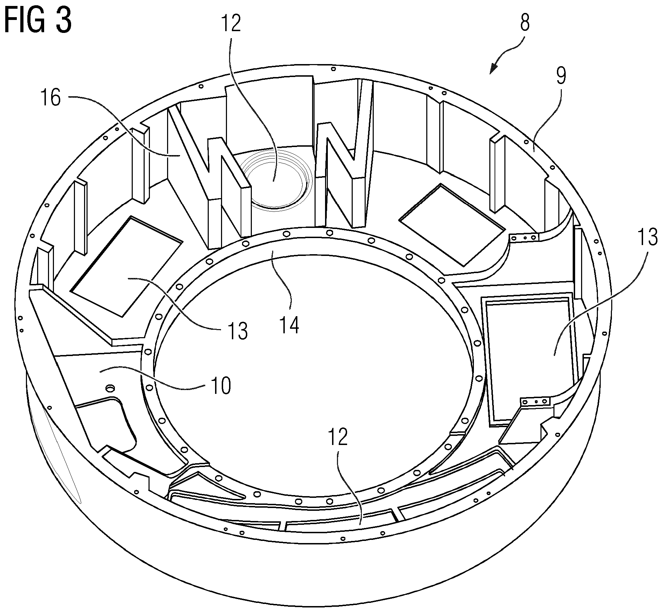

Figur 3 zeigt ein Drehgestell 8 gemäß Stand der Technik. Dieses ist ein Metallgussteil, insbesondere ist es durch Aluminium-Sandguss hergestellt. Das Drehgestell 8 weist eine Grundplatte 10 auf, die die Grundform einer runden Lochscheibe aufweist, jedoch mit Aussparungen 13 versehen ist. Am Außenumfang ist eine trommelartige, durchgehende Wand 9 vorgesehen. Diese Wand 9 weist eine relativ dicke Wandstärke von über 20 mm auf, da sie während der Rotation des Drehgestells 8 mit den rotierenden Komponenten 20 hohe Kräfte aufnehmen muss, ohne sich zu stark zu verformen, da ansonsten die Röntgenröhre 22 und die Detektoreinheit 24 ihre relative Position zueinander verlieren. Am Innenradius des Drehgestells 8 ist ein niedrigerer Vorsprung 14 vorgesehen. In Radialrichtung sind Zwischenwände 16 auf der Grundplatte 10 angeordnet, die die einzelnen Aufnahmebereiche 12 für die rotierenden Komponenten der Gantry voneinander trennen. Somit bildet das Drehgestell 8 Aufnahmebereiche 12 für rotierende Komponenten 20 des Computertomografen, welche insbesondere von den Wänden 9, 14 sowie 16 umschlossen sind. Aufgrund der Herstellung durch Sandguss ist es jedoch nicht möglich, dass die Außenwand 9 sich beispielsweise wieder nach innen wölbt oder eine Hinterschneidung bildet, obgleich dieses zur Festlegung der rotierenden Komponenten 20 hilfreich wäre. Darüber weist das Drehgestell 8 ein sehr hohes Gewicht auf.

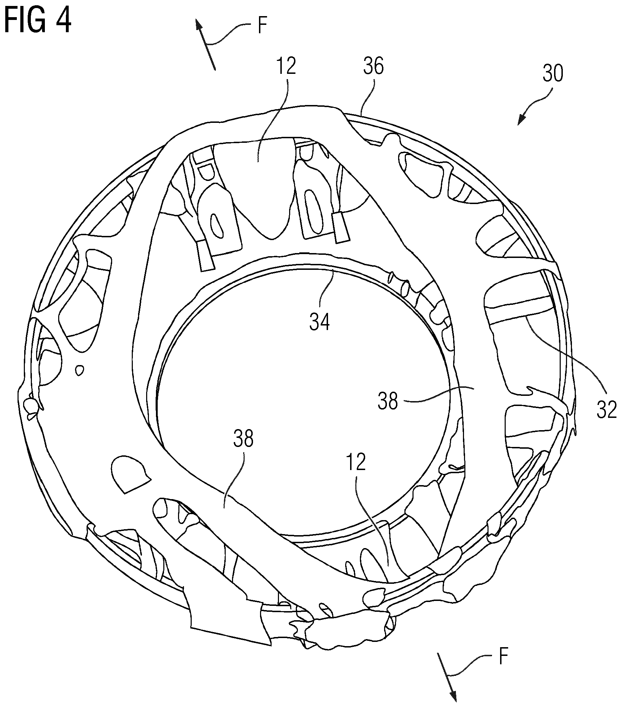

Figur 4 zeigt ein Drehgestell 30 gemäß einem Ausführungsbeispiel der der Erfindung. Dieses ist in Leichtbauweise mittels additiver Fertigung ausgeführt. Es weist einen inneren Ring 34 auf, der einen solchen Durchmesser hat, dass an ihm die Lagerung an dem Ring 7 der Gantry erfolgen kann. Ferner ist ein äußerer Ring 36 vorgesehen, an dem die fachwerkartige Struktur am Außenumfang des Drehgestell 30 befestigt ist. Zwischen dem inneren Ring 34 und dem äußeren Ring 36 verlaufen Verstrebungen 32 in unregelmäßigen Formen und Dicken. Diese Formen und Dicken wurden gemäß dem Gewicht der einzusetzenden rotierenden Komponenten 20 berechnet, um eine optimale Steifigkeit und Stabilität bei möglichst geringem Gewicht zu gewährleisten. Darüber hinaus sind am äußeren Ring 36 zwei sehnenartige Verstrebungen 38 angeordnet, die sich über die Aufnahmebereiche für die rotierenden Komponenten wölben und den äußeren Ring 36 abstützen und gegen Verformung schützen. Bei einer Rotation des Drehgestells 30 mit den eingesetzten rotierenden Komponenten 20 und hoher Drehzahl sind nämlich Kräfte in Richtung der Pfeile F zu erwarten, welche ohne die Verstrebungen 38 zu einer Verformung des Drehgestells 30, insbesondere des Rings 36 am äußeren Umfang, führen würden. Durch die Herstellung mittels additiver Fertigung ist es nun möglich, Verstärkungselemente, wie beispielsweise Verstrebungen, in zwei oder mehr in axialer Richtung voneinander beanstandeten Ebenen vorzusehen, am Beispiel der Figur 4 die Verstrebungen 32 und 38. Die dabei entstehende Form wird auch als Vogelnest bezeichnet. Das Drehgestell 30 der Figur 4 ist vollständig mittels additiver Fertigung hergestellt. Es ist jedoch auch möglich, den inneren Ring 34 und gegebenenfalls auch den äußeren Ring 36 konventionell herzustellen, und dann durch Hybridtechnik die dazwischenliegenden Elemente, insbesondere die Verstrebungen 32 und 38, mittels additiver Fertigung an die Ringe anzusetzen.

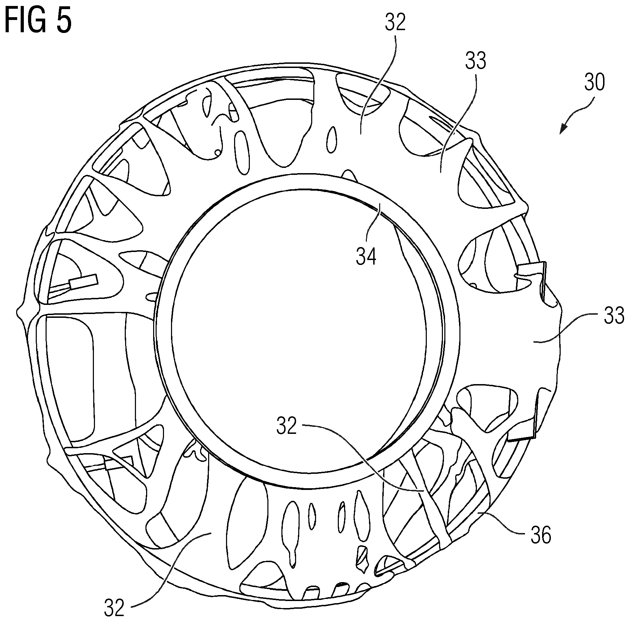

Figur 5 zeigt das Drehgestell 30 der Figur 4 von der anderen Seite. Hier sieht man besonders gut die zahlreichen Verstrebungen 32, die unregelmäßig geformt sind und dabei eine Art Fachwerk bilden, welches den inneren Ring 34 mit dem äußeren Ring 36 verbindet. Die Verstrebungen 32 sind teilweise auch flächig ausgebildet, wie beispielsweise bei 33 zu sehen.

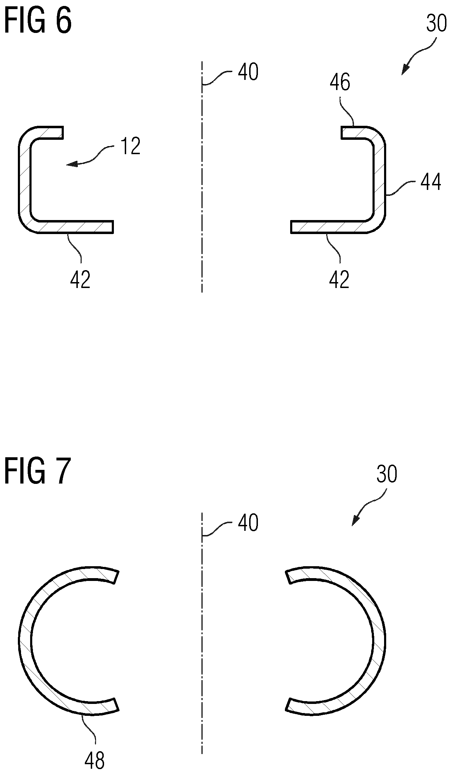

Figur 6 zeigt einen Querschnitt entlang der Achse 40 durch ein Drehgestell 30 gemäß einer anderen Ausführungsform. Dieses Drehgestell 42 hat einen Grundrahmen 42, der ungefähr die Form einer runden Lochscheibe aufweist. In Radialrichtung außen ist dann eine trommelförmige Wand 44 angesetzt, welche sich, in axialer Richtung vom Grundrahmen 42 versetzt, bei 46 wieder nach innen wölbt. Dadurch wird bei 46 eine Hinterschneidung gebildet, und eine im Aufnahmebereich 12 angeordnete rotierende Komponente 20 wird sicher gehalten. In anderen Worten kann eine rotierende Komponente 20 von zwei Seiten von Elementen des Drehgestells umgriffen werden, nämlich von dem Grundrahmen 42 und der Hinterschneidung 46. Sowohl der Grundrahmen 42 als auch der trommelartige Teil 44 und der Abschnitt 46 sind vorzugsweise im Wesentlichen aus Verstrebungen bzw. Fachwerk gebildet.

Figur 7 zeigt einen Querschnitt entlang der Achse 40 durch ein Drehgestell gemäß einer noch anderen Ausführungsform. In diesem Fall hat das Drehgestell 30 die Form eines nach innen offenen Torus oder Reifens 48. Auch hier ist die Wand 48 des Drehgestells vorzugsweise aus Verstrebungen gebildet, die insbesondere ein Fachwerk bilden.

## Patentansprüche

1. Drehgestell (30) für die Gantry (2) eines Computertomografen (1), welches Aufnahmebereiche (12) aufweist, an denen rotierende Komponenten (20, 22, 24) des Computertomografen befestigbar sind, wobei das Drehgestell (30) zumindest teilweise durch additive Fertigung hergestellt ist, **dadurch gekennzeichnet, dass** es mittels additiver Fertigung ausgebildete Hinterschneidungen (46) aufweist, wobei eine Hinterschneidung der mittels additiver Fertigung ausgebildeten Hinterschneidungen (46) dazu beiträgt, dass ein Aufnahmebereich der Aufnahmebereiche (12) von zwei Seiten von Abschnitten des Drehgestells (30) zumindest teilweise umgriffen wird.

2. Drehgestell (30) nach Anspruch 1, **dadurch gekennzeichnet, dass** es mittels additiver Fertigung ausgebildete Verstrebungen (32, 38) aufweist.

3. Drehgestell (30) nach einem der vorhergehenden Ansprüche, wobei das Drehgestell (30) um eine Achse (40) drehbar ist, wobei der Aufnahmebereich in Axialrichtung von den zwei Seiten von den Abschnitten des Drehgestells (30) umgeben ist.

4. Drehgestell (30) nach einem der vorhergehenden Ansprüche, **dadurch gekennzeichnet, dass** das Drehgestell (30) in Leichtbauweise, insbesondere mit einer mittleren Wandstärke von bis zu 20mm, ausgeführt ist.

5. Drehgestell (30) nach einem der vorhergehenden Ansprüche, **dadurch gekennzeichnet, dass** das Drehgestell (30) 20%-60%, vorzugsweise 30%-50%, weniger als ein entsprechendes Drehgestell aus Aluminiumguss gemäß Stand der Technik wiegt.

6. Drehgestell (30) nach einem der vorhergehenden Ansprüche, **dadurch gekennzeichnet, dass** es einen nicht durch additive Fertigung hergestellten Rahmen (42), sowie weitere, durch additive Fertigung hergestellte, Elemente aufweist.

7. Drehgestell (30) nach Anspruch 6, **dadurch gekennzeichnet, dass** der Rahmen (42) aus einem gewalzten Metallelement hergestellt ist.

8. Drehgestell (30) nach einem der Ansprüche 1 bis 5,
**dadurch gekennzeichnet, dass** es vollständig durch additive Fertigung hergestellt ist.

9. Drehgestell (30) nach einem der vorhergehenden Ansprüche, **dadurch gekennzeichnet, dass** es Fachwerkelemente aufweist, welche zumindest einen Aufnahmebereich auf zumindest einer Seite umschließen.

10. Drehgestell (30) nach einem der vorhergehenden Ansprüche, **dadurch gekennzeichnet, dass** es zumindest einen durchgehenden Ring aufweist (34, 36), an dem/denen Verstrebungen mittels einem additiven Fertigungsverfahren angesetzt sind.

11. Drehgestell (30) nach einem der vorhergehenden Ansprüche, **dadurch gekennzeichnet, dass** das Drehgestell (30) eine solche Steifigkeit aufweist, dass es bei einer Rotation im Betrieb des Computertomografen maximal bis zu 0,3mm, vorzugsweise bis zu 0,2mm, verformbar ist.

12. Gantry (2) für einen Computertomografen mit einem Drehgestell (30) gemäß einem der vorhergehenden Ansprüche, an welchem rotierende Komponenten (20, 22, 24) des Computertomografen (1), insbesondere eine Röntgenquelle (22) und eine Röntgendetektoreinheit (24), befestigt sind.

13. Computertomograf (1), umfassend das Drehgestell (30) nach einem der Ansprüche 1 bis 11 und/oder die Gantry (2) nach Anspruch 12.

## Claims

1. Rotating frame (30) for the gantry (2) of a computed tomography unit (1), which has receiving areas (12), to which rotating components (20, 22, 24) of the computed tomography unit can be attached, wherein the rotating frame (30) is produced at least in part using additive manufacturing, **characterised in that** it has undercuts (46) formed by means of additive manufacturing, wherein an undercut of the undercuts (46) designed by means of additive manufacturing contributes to a receiving area of the receiving areas (12) being encompassed at least in part from two sides by sections of the rotating frame (30).

2. Rotating frame (30) according to claim 1, **characterised in that** it has struts (32, 38) formed by means of additive manufacturing.

3. Rotating frame (30) according to one of the preceding claims, **characterised in that** the rotating frame (30) is rotatable about an axis (40), wherein the receiving area is encompassed in the axial direction from two sides by the sections of the rotating frame (30).

4. Rotating frame (30) according to one of the preceding claims, **characterised in that** the rotating frame (30) is lightweight in construction, in particular with an average wall thickness of up to 20 mm.

5. Rotating frame (30) according to one of the preceding claims, **characterised in that** the rotating frame (30) weighs 20%-60%, preferably 30%-50%, less than a corresponding rotating frame made of cast aluminium in accordance with the prior art.

6. Rotating frame (30) according to one of the preceding claims, **characterised in that** it has a frame (42) not produced using additive manufacturing, as well as further elements produced using additive manufacturing.

7. Rotating frame (30) according to claim 6, **characterised in that** the frame (42) is produced from a rolled metal element.

8. Rotating frame (30) according to one of claims 1 to 5, **characterised in that** it is completely produced using additive manufacturing.

9. Rotating frame (30) according to one of the preceding claims, **characterised in that** it has bracing elements which enclose at least one receiving area on at least one side.

10. Rotating frame (30) according to one of the preceding claims, **characterised in that** it has at least one continuous ring (34, 36), on which struts are positioned by means of an additive manufacturing process.

11. Rotating frame (30) according to one of the preceding claims, **characterised in that** the rotating frame (30) has a rigidity, such that during a rotation in operation of the computed tomography unit it is maximally deformable by up to 0.3 mm, preferably up to 0.2 mm.

12. Gantry (2) for a computed tomography unit with a rotating frame (30) in accordance with one of the preceding claims, to which are attached rotating components (20, 22, 24) of the computed tomography unit (1), in particular an X-ray source (22) and an X-ray detector unit (24).

13. Computed tomography unit (1), comprising the rotating frame (30) according to one of claims 1 to 11 and/or the gantry (2) according to claim 12.

## Revendications

1. Bâti (30) tournant pour le portique (2) d'un tomodensitomètre (1) assisté par ordinateur, lequel a des zones (12) d'enregistrement, auxquelles des composants (20, 22, 24) tournants du tomodensitomètre assisté par ordinateur peuvent être fixés,
dans lequel
le bâti (30) tournant est fabriqué au moins en partie par fabrication additive, **caractérisé en ce qu'**il a des contre-dépouilles (46) formées au moyen d'une fabrication additive, dans lequel une contre-dépouille, parmi les contre-dépouilles (46) formées au moyen de la fabrication additive, contribue à ce qu'une zone d'enregistrement parmi les zones (12) d'enregistrement soit prise au moins en partie de deux côtés par des parties du bâti (30) tournant.

2. Bâti (30) tournant suivant la revendication 1, **caractérisé en ce qu'**il a des entretoises (32, 38) formées au moyen d'une fabrication additive.

3. Bâti (30) tournant suivant l'une des revendications précédentes, dans lequel le bâti (30) tournant peut tourner autour d'un axe (40), dans lequel la zone d'enregistrement est entourée dans la direction axiale des deux côtés par les parties du bâti (30) tournant.

4. Bâti (30) tournant suivant l'une des revendications précédentes, **caractérisé en ce que** le bâti (30) tournant est réalisé en mode de construction légère en ayant, en particulier, une épaisseur moyenne de paroi allant jusqu'à 20 mm.

5. Bâti (30) tournant suivant l'une des revendications précédentes, **caractérisé en ce que** le bâti (30) tournant est moins pesant de 20 % à 60 %, de préférence de 30 % à 50 %, qu'un bâti tournant correspondant en fonte d'aluminium suivant l'état de la technique.

6. Bâti (30) tournant suivant l'une des revendications précédentes, **caractérisé en ce qu'**il a un cadre (42), qui n'est pas fabriqué par la fabrication additive, ainsi que d'autres éléments fabriqués par fabrication additive.

7. Bâti (30) tournant suivant la revendication 6, **caractérisé en ce que** le cadre (42) est fabriqué à partir d'un élément métallique laminé.

8. Bâti (30) tournant suivant l'une des revendications 1 à 5, **caractérisé en ce qu'**il est fabriqué entièrement par fabrication additive.

9. Bâti (30) tournant suivant l'une des revendications précédentes, **caractérisé en ce qu'**il a des éléments de treillis, qui entourent au moins d'un côté au moins une zone d'enregistrement.

10. Bâti (30) tournant suivant l'une des revendications précédentes, **caractérisé en ce qu'**il a (34, 36) au moins un anneau continu, auquel/auxquels des entretoises sont ajoutées au moyen d'un procédé de fabrication additive.

11. Bâti (30) tournant suivant l'une des revendications précédentes, **caractérisé en ce que** le bâti (30) tournant a une rigidité telle que, lors d'une rotation alors que le tomodensitomètre assisté par ordinateur est en fonctionnement, il peut se déformer au maximum jusqu'à 0,3 mm, de préférence jusqu'à 0,2 mm.

12. Portique (2) d'un tomodensitomètre assisté par ordinateur, comprenant un bâti (30) tournant suivant l'une des revendications précédentes, auquel sont fixés des composants (20, 22, 24) tournants du tomodensitomètre (1) assisté par ordinateur, en particulier une source (22) de rayons X et une unité (24) formant détecteur de rayons X.

13. Tomodensitomètre (1) assisté par ordinateur, comprenant le bâti (30) tournant suivant l'une des revendications 1 à 11, et/ou le portique (2) suivant la revendication 12.
